# EUROPEAN PATENT APPLICATION

(11) **EP 3 766 359 A1**
(43) Date of publication of application: **20.01.2021**
(21) Application number: 19768497.0
(22) Date of filing: 18.03.2019
(51) Int. Cl.: A23K 20/163, A23K 10/10, A23K 20/24, A23K 40/30, C12P 19/04, C12P 19/06, C12P 19/08, C12P 19/10

(54) **FEED ADDITIVE AND FEED**

(30) Priority: 16.03.2018 JP 2018049061
(71) Applicant: AJINOMOTO CO., INC., Chuo-ku Tokyo 104-8315 (JP)
(72) Inventor: UEHARA, Akinori, Kawasaki-shi, Kanagawa 210-8681 (JP); MAEKAWA, Mayumi, Kawasaki-shi, Kanagawa 210-8681 (JP); NAKAGAWA, Kazuki, Kawasaki-shi, Kanagawa 210-8681 (JP)
(74) Representative: Strehl Schübel-Hopf & Partner
(86) International application number: PCT/JP2019/011163
(87) International publication number: WO 2019/177172

(57) **Abstract**

The present invention provides a coated-type feed additive composition for improving body weight gain of livestock or feed conversion ratio for livestock comprising: a core containing a polysaccharide having a property of aggregating gram-negative bacteria; and a core coating agent.

## Description

### Technical Field

The present invention relates to a feed additive suitable for livestock, particularly monogastric animals, and a feed containing the additive.

### Background Art

There are various types of feed additive content that promote the growth of livestock or improve the performance of livestock production. One of the approaches to promote the growth or improve the performance of livestock production is to remove pathogenic bacteria from the body of livestock and improve the intestinal environment.

The pathogenic bacteria bind to and grow in epithelial cells of the intestinal tract of livestock. Mannose is known to block the binding of Salmonella to the epithelial cells of the intestinal tract of livestock (for example, Non Patent Literature 1). Since pathogenic bacteria die if they cannot proliferate, it can be expected that mannose will eventually eliminate pathogenic bacteria from the body by stopping the binding of pathogenic bacteria to epithelial cells. However, on the other hand, it is also known that bad bacteria assimilate mannose (for example, Patent Literature 1).

Based on these, a technique has been reported in which the feed conversion ratio is improved by condensing two molecules of mannose to form β1,4-mannobiose (Patent Literature 2). In addition, an added-to-feed composition formed by transforming monosaccharide mannose to an oligosaccharide (tri- and tetra-saccharide) has also been reported (Patent Literature 3). Polysaccharides are also used in feed because the polysaccharides are less expensive than oligosaccharides. It has also been reported that a polysaccharide containing mannose as a constituent sugar has an effect of reducing pathogenic bacteria such as Salmonella in the intestines of livestock animals (Patent Literature 4). Regarding the feed effect of polysaccharides, it has been reported by P. A. Iji et al. that high-viscosity polysaccharides have a lower body weight gain effect (also referred to as the body weight gain efficiency) than low-viscosity polysaccharides, and inhibit the development of the small intestine (Non Patent Literature 2).

Moreover, a feed additive has been reported which is characterized by containing an α-mannobiose-containing composition produced by allowing a natural product containing mannan to react with β-mannosidase and α-mannosidase sequentially or simultaneously (Patent Literature 5). Patent Literature 5 reports that the colonization of Salmonella in the animal body can be suppressed and Salmonella can be efficiently excreted out of the body. However, it is necessary to prepare β-mannosidase and α-mannosidase in addition to mannan in the production process. Patent Literature 5 also reports the finding that manno-oligosaccharides and mannose-based polysaccharides having a molecular weight higher than that of mannobiose have a small effect of inhibiting Salmonella colonization of the gut (paragraph number 0005 and the like).

### Citation List

### Patent Literatures

Patent Literature 1: International Publication No. WO2011/027753
Patent Literature 2: International Publication No. WO2011/027753
Patent Literature 3: U.S. Patent Application Publication No. 2009/0186852
Patent Literature 4: U.S. Patent No. 6126961
Patent Literature 5: Japanese Patent No. 4294302

### Non Patent Literatures

Non Patent Literature 1: B. A. OYOFO et al., Poultly Science (1989) vol. 68, 1357
Non Patent Literature 2: P. A. Iji et al., Animal feed science and technology (2001) vol. 8(3-4), 175-188

### Summary of Invention

### Problems to be solved by the invention

Therefore, an object of the present invention is to provide a feed additive containing a polysaccharide, which can exhibit a body weight gain effect also on healthy livestock and is economical.

### Means for solution of the problems

As a result of diligent studies by the present inventors, it has been found that the body weight gain effect and feed conversion ratio can be enhanced by providing a chicken with a feed coated with a polysaccharide having an effect of aggregating gram-negative bacteria. Based on this finding, the present inventors have completed an invention relating to a feed additive which can enhance the body weight gain effect of livestock when coated with a polysaccharide having an effect of aggregating gram-negative bacteria even if the polysaccharide does not contain mannose as a constituent sugar. Specifically, the following inventions are provided.
1. A coated-type feed additive composition for improving body weight gain of livestock or feed conversion ratio for livestock comprising:
   a core containing a polysaccharide having a property of aggregating gram-negative bacteria; and
   a core coating agent.
2. A coated-type feed additive composition for improving body weight gain of livestock or feed conversion ratio for livestock according to 1 describes above, wherein
   the polysaccharide is a polysaccharide containing, as a constituent sugar, at least one selected from the group consisting of mannose (Man), glucose (Glc), galactose (Gal), glucuronic acid (GlcA), mannuronic acid, and guluronic acid.
3. A coated-type feed additive composition for improving body weight gain of livestock or feed conversion ratio for livestock according to 1 describes above, wherein
   the polysaccharide is at least one selected from the group consisting of pullulan, xanthan gum, guar gum, chitosan, carrageenan, arabic gum, pectin, carboxymethyl cellulose, methyl cellulose, hydroxypropyl cellulose, hydroxypropyl methylcellulose, dextran, chondroitin, tara gum, locust bean gum, alginates (sodium salts, potassium salts, calcium salts, or ammonium salts), alginic acid esters, and mixtures thereof.
4. A coated-type feed additive composition for improving body weight gain of livestock or feed conversion ratio for livestock according to 1 describes above, wherein
   the polysaccharide is a culture product of Xanthomonas campestris, Aureobasidium pullulans, Lipomyces starkeyi, or brown algae (Phaeophyta).
5. A coated-type feed additive composition for improving body weight gain of livestock or feed conversion ratio for livestock according to any one of 1 to 4 describes above, wherein
   the coating agent contains hydrogenated vegetable oil.
6. A coated-type feed additive composition for improving body weight gain of livestock or feed conversion ratio for livestock according to 5 describes above, wherein
   the hydrogenated vegetable oil is a hydrogenated rapeseed oil, hydrogenated linseed oil, hydrogenated safflower oil, hydrogenated sunflower oil, hydrogenated soybean oil, hydrogenated corn oil, hydrogenated peanut oil, hydrogenated cottonseed oil, hydrogenated sesame oil, hydrogenated rice oil, hydrogenated olive oil, hydrogenated palm oil, hydrogenated palm kernel oil, or hydrogenated coconut oil.
7. A coated-type feed additive composition for improving body weight gain of livestock or feed conversion ratio for livestock according to any one of 1 to 6 describes above, wherein
   the polysaccharide is pullulan, xanthan gum, or arabic gum, and
   the coating agent is hydrogenated rapeseed oil and/or shellac.
8. A coated-type feed additive composition for improving body weight gain of livestock or feed conversion ratio for livestock according to 7 describes above, wherein
   a coating layer formed by the coating agent has a two-layer structure including a layer formed of hydrogenated rapeseed oil and a layer formed of shellac.
9. A coated-type feed additive composition for improving body weight gain of livestock or feed conversion ratio for livestock according to 8 describes above, wherein
   the layer formed of shellac is in contact with the core, and the layer formed of hydrogenated rapeseed oil is formed thereon.
10. A coated-type feed additive composition for improving body weight gain of livestock or feed conversion ratio for livestock according to any one of 1 to 9 describes above, wherein
   the core further contains a salt of a divalent inorganic cation.
11. A coated-type feed additive composition for improving body weight gain of livestock or feed conversion ratio for livestock according to 10 describes above, wherein
   the salt of a divalent inorganic cation is a salt of Mg, Ca, or Fe.
12. A coated-type feed additive composition for improving body weight gain of livestock or feed conversion ratio for livestock according to 11 describes above, wherein
   the salt of a divalent inorganic cation is a salt of Ca.
13. A feed comprising a coated-type feed additive composition for improving body weight gain of livestock or feed conversion ratio for livestock according to any one of 1 to 12 describes above.
14. Use of a coated agent comprising a core containing a polysaccharide having a property of aggregating gram-negative bacteria and a core coating agent, as an improver for body weight gain of livestock or feed conversion ratio for livestock.

### Advantageous Effects of Invention

The coated-type feed additive of the present invention makes it possible to provide a feed additive containing a polysaccharide, which can exhibit a body weight gain effect also on healthy livestock and is economical.

### Brief Description of Drawings

Fig. 1A illustrates the change over time of the optical density of an Escherichia coli suspension containing Ca²⁺ alone or 0.5% pullulan in the presence or absence of Ca²⁺
Fig. 1B illustrates the change over time of the optical density of an Escherichia coli suspension containing 0.05% pullulan in the presence or absence of Ca²⁺.
Fig. 1C illustrates the change over time of the optical density of an Escherichia coli suspension containing 0.05% xanthan gum in the presence or absence of Ca²⁺.
Fig. ID illustrates the change over time of the optical density of an Escherichia coli suspension containing 0.005% xanthan gum in the presence or absence of Ca²⁺.
Fig. 2 illustrates the change over time in the dissolution rate of xanthan gum from a one-layer coated-type feed additive (XG-1) and a two-layer coated-type feed additive (XG-2) under gastric and intestinal juice conditions.
Fig. 3 illustrates the change over time in the dissolution rate of arabic gum from a two-layer coated-type feed additive (coated arabic gum) under gastric and intestinal juice conditions.

### Description of Embodiments

The coated-type body weight gain effect or feed conversion ratio improver of the present invention (hereinafter collectively referred to as the "coated-type feed additive") is composed of a core containing a polysaccharide having a property of aggregating gram-negative bacteria and a coating agent thereof.

### [Core]

The polysaccharide used in the present invention has a property of aggregating gram-negative bacteria such as Escherichia coli. The polysaccharide may be used alone or in combination of two or more kinds.

The aggregation ability can be judged by whether or not the optical density of the suspension containing the culture of gram-negative bacteria is lowered by the addition of a polysaccharide. The suspension before the addition of a polysaccharide is white and turbid, but when a polysaccharide having an aggregation ability is present, gram-negative bacteria aggregate and settle down, and the transparency of the suspension increases. Since the bacterial cells in the suspension without the addition of a polysaccharide spontaneously settle down, it can be judged that the aggregation effect by the polysaccharide has been recognized when the polysaccharide is added to the suspension and the decrease is exceeded in the optical density of the suspension due to the spontaneous sedimentation of the bacterial cells over a certain period of time, for example, 12 to 24 hours. The certain period of time mentioned here can be appropriately adjusted by those skilled in the art in consideration of the time from when the feed reaches the stomach to when it passes through the intestine, depending on the type of livestock to be administered with the feed. In the present specification, in consideration of the error of the experimental system, it is judged that the aggregation effect has been recognized when the decrease in optical density due to spontaneous sedimentation is decreased by at least twice the optical density of the suspension due to spontaneous sedimentation of the bacterial cells for a certain aggregation time. The aggregation time mentioned here refers to the above-mentioned certain period of time or the time X at which the optical density of the suspension is measured with time and the plateau is reached. For example, when the optical density reaches the plateau at the aggregation time X, the difference of the suspension between the optical density at time 0 and the optical density at time X is defined as "ΔODsample [0, X]," and the difference of the negative control between the optical density at time 0 and the optical density at time X is defined as "ΔODnc [0, X]." When ΔODsample [0, X] ≥ ΔODnc [0, X] × 2, it is determined that there is an aggregation effect.

In addition, the aggregation rate can be obtained by dividing the difference between ΔODsample [0, X] and ΔODnc [0, X] by the aggregation time which is the time when the plateau is reached. Even before the plateau is reached, the aggregation rate can be determined by using the specific time after the addition of the polysaccharide as a guide. This "specific time" corresponds to the time from when the feed reaches the stomach of the livestock until when it passes through the intestine, and differs depending on the type of livestock. For example, in the case of chicken, it can be 4 hours.

The present inventors comprehensively investigated polysaccharides, and have found that excellent aggregation ability is observed in a polysaccharide having, as a constituent sugar, at least one selected from the group consisting of mannose (Man), glucose (Glc), galactose (Gal), glucuronic acid (GlcA), mannuronic acid, and guluronic acid. Such a polysaccharide includes pullulan (PLN), xanthan gum (XG), guar gum, carrageenan (especially κ-carrageenan), arabic gum, carboxymethyl cellulose (CMC), methyl cellulose, hydroxypropyl cellulose, hydroxypropyl methylcellulose, dextran, chondroitin, alginates (such as sodium salts, potassium salts, calcium salts, and ammonium salts), alginic acid esters (such as alginic acid propyl alcohol), and the like. Therefore, preferably, the polysaccharide having a property of aggregating gram-negative bacteria has, as a constituent sugar, at least one selected from the group consisting of mannose (Man), glucose (Glc), galactose (Gal), and glucuronic acid (GlcA).

The present inventors have also found that even a polysaccharide that does not have the above-mentioned predetermined saccharide as a constituent sugar has excellent aggregation ability. Such a polysaccharide includes chitosan and pectin.

As described above, preferably, the polysaccharide having a property of aggregating gram-negative bacteria is also at least one selected from the group consisting of pullulan, xanthan gum, guar gum, carrageenan (especially κ-carrageenan), arabic gum, chitosan, pectin, carboxymethyl cellulose, methyl cellulose, hydroxypropyl cellulose, hydroxypropyl methylcellulose, dextran, chondroitin, tara gum, locust bean gum, alginates (such as sodium salts, potassium salts, calcium salts, and ammonium salts), alginic acid esters (such as alginic acid propyl alcohol), and mixtures thereof. Arabic gum, pullulan, or xanthan gum is more preferable. Arabic gum or xanthan gum is further preferable. Arabic gum is particularly preferable.

The purity of the polysaccharide used in the present invention is not limited as long as the desired effect is obtained. For example, a polysaccharide obtained by being produced by a microorganism can be used together with the fermentation broth. The solid content in the fermentation broth can be obtained by freeze-drying, or the fermentation broth can be obtained as a solid content by spray granulation. As a microorganism that produces a polysaccharide, for example, galactoglucomannan (GGM) produced by Lipomyces starkeyi can be obtained by the methods described in Japanese Patent Application Publication No. Hei 7-298873 and Japanese Patent Application Publication No. Hei 9-131199. Preferably, the polysaccharide having a property of aggregating gram-negative bacteria is also a culture product of Xanthomonas campestris, Aureobasidium pullulans, or Lipomyces starkeyi. As the polysaccharide used in the present invention, a secreted product or extracted product from plants, algae, crustaceans, or fish can be used as it is. Regarding algae, among brown algae (Phaeophyta), for example, a product containing alginic acid produced by Macrocystis pyrifera or Laminaria hyperborea can also be used. As the polysaccharide used in the present invention, a commercially available product can be used as it is. The culture product of arabic gum, pullulan, or xanthan gum is more preferable. The culture product of arabic gum or xanthan gum is further preferable.

The viscosity of the polysaccharide used in the present invention is not limited. For reference, the viscosities of polysaccharides are presented in the table below (the temperature is 25°C, and the concentration is described in the table). It has been reported that high-viscosity polysaccharides are inferior to low-viscosity polysaccharides in body weight gain effect (P. A. Iji et al., Animal feed science and technology (2001) vol. 8(3-4) pp 175-188 cited above), but the present inventors have found that, when a polysaccharide is coated with a coating agent regardless of the viscosity, it is possible to exhibit a body weight gain effect equal to or higher than that of a low-viscosity polysaccharide without coating.

| Name | mPa·s @ 25°C | % | reference source |
|---|---|---|---|
| Arabic Gum | 50-150 | 25.0 | http://www.foodchemadditives.com/jp/Thickeners/Acacia-Gum.html |
| Guar Gum | 1000 to 1500 | 1.0 | http://www.mfc.co.jp/product/pdf/rg100.pdf |
| k-Carrageenan | 5-25 | 0.3 | http://www.sigmaaldrich.com/catalog/product/sigma/22048?lang=ja&region =JP |
| Xanthan Gum | 1200 to 1600 | 1.0 | http://www.mfc.co.jp/product/pdf/xg400.pdf |
| Pullulan | 15 to 180 | 10.0 | http://www.tcichemicals.com/eshop/ja/jp/commodity/P0978/ |
| CMC | 100 to 2000 | 1.0 | http://www.daicelfinechem.jp/business/wspdiv/cmc.html |
| Chitosan | 10 to 100 | 0.5 | http://www.koyochemical.jp/product/chitosan |
| Chondroitin | 5 to 10 | 24.0 | https://www.ncbi. nlm.nih.gov/pmc/articles/PMC2290215/pdf/tjp0540-0271. pdf |
| Pectin | 300 | ? | https://www.buerkle.de/files_pdf/wissenswertes/viscosity _en_2016.pdf |
| Glucuronate | N.D. | N.D. | N.D. |
| Gellan Gum | 100 | 1.0 | https://rd.sprincer.com/content/pdf/10.1007%2Fs003960050446.pdf |
| Alginic acid | 20 to 900 | 1.0 | http://www.kimica.jp/products/detail/ |
| Locust bean gum | 1000 to 1200 | 1.0 | http://www.tatourui.com/about/type/03_locust bean.html |
| Tara Gum | 1000 to 1200 | 1.0 | http://www.mfc.co.jp/product/pdf/mt120.pdf |
| Sterculia Gum (Karaya Gum) | 120 to 400 | 0.5 | https://books.google.cojp/books?id=AERPFV4MuwUC&pg=PT221&lpg= PT221&dq=karaya+gum+viscosity+%EF%BD%8DPa%E3%83%BBs&sou rce=bl&ots=M3RVtQhiAy&sig=mR9RWUex60E414yqbxkYA-lklSw&hl=j a&sa=X&ved=0ahUKEwjPrcOWovTXAhWGnJQKHZtoCW cQ6AEIPzAD #v=onepage&q=karaya%20gum%20viscosity%20%EF%BD%8DPa%E3%8 3%BBs&f=false |
| gum tragacanth | 3500 | 1 | https://books.google.co.jp/books?id=AERPFV4MuwUC&pg=PT214&lpg= PT214&dq=gum+tragacanth+viscosity+%EF%BD%8DPa%E3%83 %BBs& source=bl&ots=M3RVtQiazy&sig=plM_KEQkax9l2IcjSuzVYxqG90M&hl =ja&sa=X&ved=0ahUKEwjoybXxovTXAhWCJZQKHbqaBqAQ6AEIJzA A#v=onepage&q=gum%20tragacanth%20viscosity%20%EF%BD%8DPa% E3%83%BBs&f=false |
| Sodium alginate | 300-400 | 1 | Reagent bottle grade display 2% 20°C |
| Methylcellulose | 100 (mm2/s) | 2 | Reagent bottle display Methylcellulose 100 (mm2/s)* 2% 20°C |
| Hydroxypropylcellulose | 150 to 400 | 2 | Reagent bottle grade display 2% 20°C |
| Hydroxypropylmethylcellulose | up to 4000 | 2 | Product information 2% 20°C https://www.sigmaaldrich.com/catalog/product/sial/h3785?lang=ja&region= JP |
| Dextrans | 400 | ? | Dextran 70 https://www.druginformer.com/search/side_effect/dextran+70%3B+hyprom ellose+2910+(4000+mpa.s) |

The core may contain divalent metal ions. The divalent metal ions include ions of magnesium, calcium, iron, zinc, and the like. Among these, calcium ions are particularly preferable. The divalent metal ions may be in the form of a compound with a halogen such as chlorine; an organic acid such as citric acid, gluconic acid, and succinic acid; an inorganic acid such as carbonic acid and sulfuric acid. Examples thereof include calcium chloride, iron citrate, zinc carbonate, and magnesium sulfate. Among these, calcium chloride is particularly preferable.

The amount of divalent metal ions blended is preferably 0.2 to 3 times, more preferably 0.3 to 3 times, and further preferably 0.3 to 2 times the weight of the polysaccharide in the form of a compound with chlorine or the like.

The core may contain an excipient. The excipient is not particularly limited as long as it is one commonly used for improving shape formation, and examples thereof include calcium carbonate, silicon dioxide, calcium silicate, zeolite, sorbitol, corn starch, talc, yeast bentonite, rice husk, liquid paraffin, polysaccharides other than polysaccharides having a property of aggregating gram-negative bacteria, monosaccharides, and disaccharides. When the core contains an excipient, the amount of the excipient is usually preferably 0.1 to 100 parts by mass based on 100 parts by mass of the core.

The core may also contain any additives that may be included in feed. When the core contains an optional additive, the amount of the optional additive is usually preferably 0.1 to 100 parts by mass based on 100 parts by mass of the core.

### [Coating agent]

The coating agent is a substance capable of forming an enteric coating and can be used without particular limitation as long as it is a substance safe for livestock to ingest. The coating agent may be used alone or in combination of two or more kinds. From the viewpoint of easy handling and economical efficiency, the coating agent is preferably hydrogenated vegetable oil, or shellacs, zein, hydroxypropyl methylcellulose, maltitol, and the like which are substances commonly used as tablet coating agents. The hydrogenated vegetable oil includes hydrogenated oils of rapeseed oil, linseed oil, safflower oil, sunflower oil, soybean oil, corn oil, peanut oil, cottonseed oil, sesame oil, rice oil, olive oil, palm oil, palm kernel oil, or coconut oil. Preferably, the hydrogenated vegetable oil is a hydrogenated oil of rapeseed oil, linseed oil, safflower oil, sunflower oil, soybean oil, corn oil, peanut oil, cottonseed oil, sesame oil, rice oil, olive oil, palm oil, palm kernel oil, or coconut oil. Among them, the coating agent is preferably hydrogenated rapeseed oil and shellac. The layer of hydrogenated rapeseed oil is preferable because it can dissolve the core in a short period of time. The layer of shellac is preferable because it can dissolve the core with neutral to alkaline (after passing the stomach).

The coating agent is in an amount of preferably 5 to 90% by mass, and more preferably 20 to 30% by mass, based on the total mass of the coated-type feed additive of the present invention. The coating agent may also contain any additives that may be included in feed.

The coating may be a single layer or multiple layers of two or more layers. The multi-layer coating is preferable because it is easier to control the dissolution rate in the body. In particular, it is preferable that the outermost layer is a layer of hydrogenated rapeseed oil and the innermost layer in contact with the core is a layer of shellac, because the coating agent dissolves in the intestine rather than in the stomach.

The dissolution rate of the coated-type feed additive of the present invention in gastric juice is desirably less than 60%, and the dissolution rate in intestinal juice is desirably 70% or more. For the purpose of achieving such a dissolution rate, it can be adjusted by forming a two-layer membrane or a multi-layer membrane, or by controlling the type of coating agent and the membrane thickness for each layer.

It is particularly preferable that the polysaccharide is pullulan, xanthan gum, or arabic gum, and the coating agent is at least one selected from the group consisting of hydrogenated rapeseed oil and shellac.

It is particularly preferable that the polysaccharide is xanthan gum or arabic gum, and the coating agent is at least one selected from the group consisting of hydrogenated rapeseed oil and shellac.

It is particularly preferable that the polysaccharide is arabic gum, and the coating agent is at least one selected from the group consisting of hydrogenated rapeseed oil and shellac.

It is particularly preferable that the polysaccharide is arabic gum, and the coating agent includes two layers of hydrogenated rapeseed oil and shellac. In this case, it is further preferable that the layer formed of shellac is in contact with the core, and the layer formed of hydrogenated rapeseed oil is formed thereon.

It is particularly preferable that the core contains arabic gum and Ca²⁺, and the coating agent includes two layers of hydrogenated rapeseed oil and shellac. In this case, it is further particularly preferable that the amount of Ca²⁺ blended is 0.3 to 2 times the weight of the polysaccharide in the form of a compound. In particular, it is most preferable that the layer formed of shellac is in contact with the core, and the layer formed of hydrogenated rapeseed oil is formed thereon.

### [Coating Method]

The method of coating the core is not particularly limited, and for example, it is possible to obtain a coated-type feed additive by spraying a coating agent in a liquid state, heated to a temperature higher than the melting point, while allowing the powdered or granular core to flow with a commercially available fluidized bed spray granulator. The coated-type feed additive obtained from powdered or granular polysaccharides has a size of preferably about 0.05 to 5 mm because handling is easy. In addition, the temperature for heating the coating agent is not particularly limited as long as it is equal to or higher than the melting point of the coating agent, but it is preferably higher than the melting point of the coating agent by about 5°C to 15°C.

### [Feed]

The coated-type feed additive of the present invention can be given to livestock as it is, or can be used as a feed together with an excipient or diluent such as corn, soybean flour, rice bran, fish meal, or brewer's yeast. The feed of the present invention may also contain any additives that may be included in feed. The feed of the present invention is suitable for continuous daily intake. The feed intake varies depending on the size of livestock. For example, in the case of chickens, it is desirable that the core is fed at a daily intake of about 1 to 200 ppm and preferably 10 to 100 ppm, based on the feed other than the coated-type feed additive. Note that, in the present specification, "ppm" means "ppm by mass."

In the present specification, the term "livestock" refers to creatures that are bred by humans. Specific examples include ruminants such as cows, sheep, and goats, and monogastric animals such as horses, pigs, chickens, dogs, and fish. It is particularly preferable to give the feed of the present invention to monogastric animals.

The method of feeding the coated-type feed additive of the present invention is not particularly limited.

### Examples

### Example 1-1: Polysaccharide Screening 1

Escherichia coli MG1655 was cultured on an LB plate at 37°C for 24 hours, and the obtained culture was suspended in 4 ml of physiological saline. The optical density (OD) of the suspension was adjusted to about 2.0. This suspension, in an amount of 0.5 ml, was added to a vial, to which 5 ml of each polysaccharide solution presented in Table 1 was added, followed by reaction of E. coli with each polysaccharide. The effect of each polysaccharide agglomerating E. coli MG1655 was evaluated by allowing the vial to stand at 36°C and automatically measuring the optical density continuously.

First, a polysaccharide exhibiting a decrease in optical density by at least twice the decrease in optical density due to spontaneous sedimentation of a suspension without addition of polysaccharide after 12 hours was judged to be a polysaccharide having an aggregation effect. In Table 1, gellan gum and alginic acid are polysaccharides for which no aggregation effect was observed. Next, with the aggregation time denoted by X, the aggregation rates were compared using the following formula. Note that the aggregation time X was defined as the time from when the suspension was combined with polysaccharide until the plateau was reached. When aggregation occurred but the plateau was not reached, the aggregation rate up to 4 hours after administration of the polysaccharide to the suspension was calculated. Note that the "4 hours" is assumed to be the time from when the feed reaches the stomach of the chicken until when it passes through the intestine. The optical density was measured at OD 660 nm using Biophoto-recorder TVS062CA manufactured by ADVANTEC.

### <Formula 1> Max. Aggregation Rate (/h) = (ΔODsample [0, X] - ΔODnc [0, X]) / X

Note that the "ΔODsample [0, X]" means the difference of the suspension prepared above between the optical density at time 0 and the optical density at time X, and the "ΔODnc [0, X]" means the difference of the negative control between the optical density at time 0 and the optical density at time X.

The aggregation rate determined for each polysaccharide is presented in Table 1 together with the reaction time ("RT").

Note that the polysaccharides and derivatives thereof used are all commercial products. The producing bacteria of xanthan gum and pullulan are Xanthomonas campestris and Aureobasidium pullulans, respectively.

**[Table 1]**

| | Viscosity | | Conc.(%) | RT(hr) | Aggregation Rate (/h) | constituent sugar | | | | |
|---|---|---|---|---|---|---|---|---|---|---|
| | mPa·s @ 25°C | % | | | | Man | Glc | Gal | GlcA | Others |
| Arabic Gum | 50-150 | 25.0 | 1.00 | 4.4 | 0.36 | | | 3 | 1 | 4 |
| Guar Gum | 1000 to 1500 | 1.0 | 0.10 | 3.0 | 0.60 | 2 | | 1 | | |
| k-Carrageenan | 5-25 | 0.3 | 0.10 | 3.5 | 0.39 | | | 1 | | |
| Xanthan Gum | 1200 to 1600 | 1.0 | 0.05 | 6.6 | 0.23 | 2 | 2 | | 1 | |
| Pullulan | 15 to 180 | 10.0 | 0.50 | 3.3 | 0.45 | | ○ | | | |
| CMC | 100 to 2000 | 1.0 | 0.10 | 4.3 | 0.36 | | ○ | | | |
| Chitosan | 10 to 100 | 0.5 | 0.05 | 4.5 | 0.31 | | | | | ○ |
| Chondroitin | 5 to 10 | 24.0 | 1.00 | 2.0 | 0.79 | | | | 1 | 1 |
| Pectin | 300 | ? | 0.05 | 2.5 | 0.68 | | | | | ○ |
| Glucuronate | N.D. | N.D. | 0.10 | 2.8 | 0.55 | | | | ○ | |
| Gellan Gum | 100 | 1.0 | 0.05 | ∞ | - | | 2 | | 1 | 1 |
| Alginic acid | 20 to 900 | 1.0 | 0.01/0.1 | ∞ | - | | | | | 2 |
| Lucust bean Gum | 1000 to 1200 | 1.0 | 0.50 | 7.0 | 0.25 | 4 | | | 1 | |
| Tara Gum | 1000 to 1200 | 1.0 | 0.05 | 6.3 | 0.30 | 3 | | | 1 | |
| Sterculia Gum (Karaya Gum) | 120-400 | 0.50 | 0.05 | 6.3 | 0.08 | | | ○ | | ○ |
| gum tragacanth | 3500.00 | 1.00 | 0.10 | 3.3 | 0.58 | | | ○ | | ○ |
| Sodium alginate | 300-400 | 1.00 | 0.05 | 3.0 | 0.70 | | | | | ○ |
| Methylcellulose | 100 mm2/s | 2.00 | 0.05 | 3.0 | 0.57 | ○ | | | | ○ |
| Hydroxypropylcellulose | | 150 to 400cP | 2.00 | 0.05 | 4.8 | 0.38 | | ○ | | |
| Hydroxypropylmethylcellulose | | up to 4000cP | 2.00 | 0.10 | 3.3 | 0.53 | | ○ | | |
| Dextrans | | 400 | ? | 0.50 | 7.8 | 0.13 | | ○ | | |

| | | | | | | | | | | |
|---|---|---|---|---|---|---|---|---|---|---|
| Man: mannose, Glc: glucose, Gal: galactose, GlcA: glucuronic acid ∞: No aggregation "○" in the entries of constituent sugar indicate substituted/non-substituted constituent units. The values in the entries indicate the number of each sugar molecule contained in the structural unit. | | | | | | | | | | |

### Example 1-2: Polysaccharide Screening 2

Various saccharides presented in Table 2 were evaluated by the aggregation inhibition test described in Test Example 1 of Japanese Patent No. 4294302 (aggregation inhibition minimum concentration test) and the aggregation test of Example 1-1 of the present specification.

Mannan sugar chains are present on the surface of epithelial cells of the intestinal tract, and pathogenic bacteria bind to the sugar chains. According to the description of Test Example 1, the aggregation inhibition test of Japanese Patent No. 4294302 can be understood as a test of evaluating whether or not the saccharides presented in Table 2 are bound to the pathogenic bacteria (Salmonella) by the presence or absence of aggregation of Salmonella, on the premise that the mannan sugar chains present on the yeast surface are considered to be mannan sugar chains present on the surface of epithelial cells of the intestinal tract, the pathogenic bacteria cannot bind to the sugar chains on the yeast surface when the saccharides presented in Table 2 are bound to the sugar chain binding site in the pathogenic bacteria, and it is possible as a result to inhibit pathogenic bacteria from invading the body of the livestock. Specifically, according to the aggregation inhibition test, it can be evaluated that, if Salmonella does not agglomerate in the presence of the saccharides presented in Table 2, the saccharides can block the binding of Salmonella to the epithelial cells of the intestinal tract. The presence of an aggregation inhibition effect was judged according to the description in paragraph 0019 of Test Example 1 of the patent publication.

On the other hand, the aggregation test of Example 1-1 of the present specification is a test of evaluating whether or not the polysaccharides presented in Table 1 agglomerate pathogenic bacteria (Escherichia coli), on the hypothesis that the pathogenic bacteria, which are dispersed by being repelled from one another by their surface charge, are agglomerated due to the weakening of repulsive force resulting from the presence of polysaccharide, so that it is possible to prevent pathogenic bacteria from invading the body of the livestock. Similarly to the case described in Example 1-1, a saccharide exhibiting a decrease in optical density by at least twice the decrease in optical density due to spontaneous sedimentation of a suspension without addition of saccharide after 12 hours was judged to be a saccharide having an aggregation effect.

Since both Salmonella and Escherichia coli belong to gram-negative bacteria, both tests are common in that the test substance evaluates the activity of recognizing gram-negative bacteria.

The substances exhibiting an aggregation inhibition effect in the aggregation inhibition test were marked with ○, while substance exhibiting an aggregation effect in the aggregation test of the present specification was marked with ○. The results are also presented in Table 2.

It can be seen from Table 2 that there are substances that exhibit no aggregation ability in the aggregation test even though they have a gram-negative bacteria recognizing activity screened in the aggregation inhibition test, and that they are different evaluation systems. For example, pullulan, which exhibited no inhibition effect in the aggregation inhibition test, exhibited an aggregation effect in the aggregation test, while methyl α-D-mannopyranoside, gellan gum, and alginic acid, which exhibited an inhibition effect in the aggregation inhibition test, did not exhibit the aggregation effect in the aggregation test.

**[Table 2]**

| No. | Material | Aggregation Inhibition Test of Japanese Patent No. 4294302 | Aggregation Test of Present Specification |
|---|---|---|---|
| 1 | D-(+)-Mannose | - | × |
| 2 | N-Acetylglucosamine | × | × |
| 3 | L-Arabinose | × | × |
| 4 | meso-Erythritol | × | × |
| 5 | Fructose | × | × |
| 6 | D-(+)-Galactose | × | × |
| 7 | D-Glucuronic Acid | ○ | ○ |
| 8 | D-Glucose | × | × |
| 9 | D-Mannitol | × | × |
| 10 | L-Rhamnose | × | × |
| 11 | D-(-)-Salicin | × | × |
| 12 | Xylitol | × | × |
| 13 | D-(+)-Xylose | × | × |
| 14 | D-(+)-Glucosamine | × | × |
| 15 | D-(+)-Cellobiose | × | × |
| 16 | Lactose | × | × |
| 17 | D-(+)-Maltose | × | × |
| 18 | Sucrose | × | × |
| 19 | D-(+)-Trehalose | × | × |
| 20 | Maltitol | × | × |
| 21 | D-(+)-Raffinose | ○ | × |
| 22 | D-(+)-Maltotriose | ○ | × |
| 23 | Maltoheptuose | ○ | × |
| 24 | Starch (Soluble) | × | × |
| 25 | α-cyclodextrin | × | × |
| 26 | β-cyclodextrin | × | × |
| 27 | Chitosan (soluble?) | × | ○ |
| 28 | Chondroitin | × | ○ |
| 29 | Xanthan Gum | ○ | ○ |
| 30 | Pullulan | × | ○ |
| 31 | Gellan Gum | ○ | × |
| 32 | Carrageenan | ○ | ○ |
| 33 | Pectin | ○ | ○ |
| 34 | Alginic acid | ○ | × |
| 35 | Curdlan | × | × |
| 36 | Arabic Gum | ○ | ○ |
| 37 | Guar Gum | ○ | ○ |
| 38 | CMC | × | ○ |
| 39 | Methyl α-D-mannopyranoside | ○ | × |
| 40 | D-(+)-Melezitose | × | × |
| 41 | Actigen(alltech) | ○ | × |
| 42 | Yeast Dry Cell (Ajinomoto) | - | × |
| 43 | Yeast -Glucan (Ajinomoto) | - | × |
| 44 | Sterculia Gum (Karaya Gum) | - | ○ |
| 45 | gum tragacanth | - | ○ |
| 46 | Sodium alginate | - | ○ |
| 47 | Methylcellulose | - | ○ |
| 48 | Hydroxypropylcellulose | - | ○ |
| 49 | Hydroxypropylmethylcellulose | - | ○ |
| 50 | Dextrans | - | ○ |

### Example 1-3: Aggregation ability in Presence of Divalent Metal Ions

The OD was measured in the same manner as in Example 1-1 except that CaCl2 was added to each of the polysaccharide solutions having various concentrations so as to be 10 mM, and the combined effect of the polysaccharide and CaCl2 was verified.

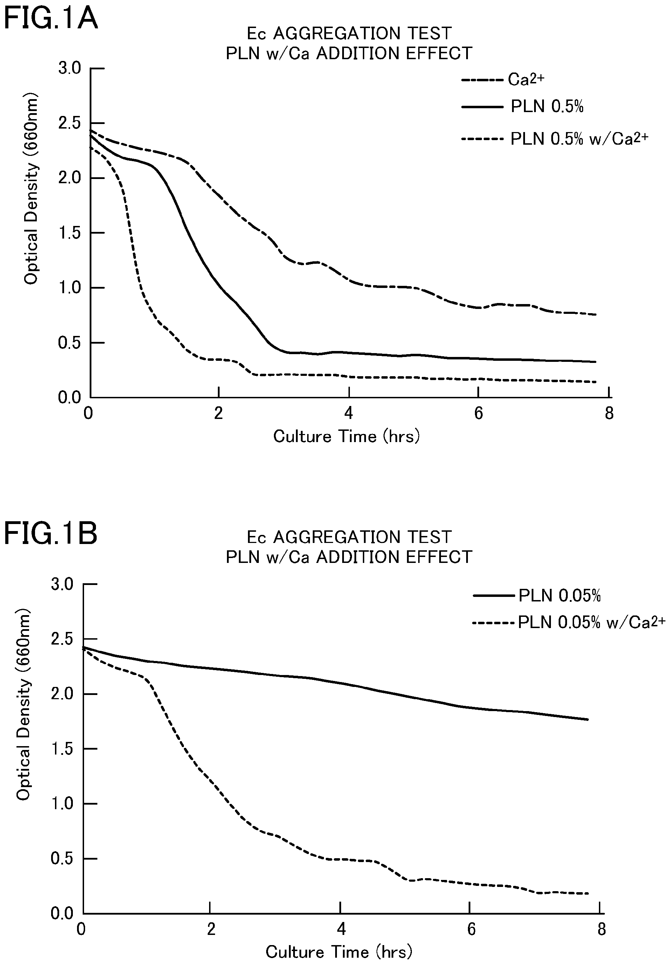

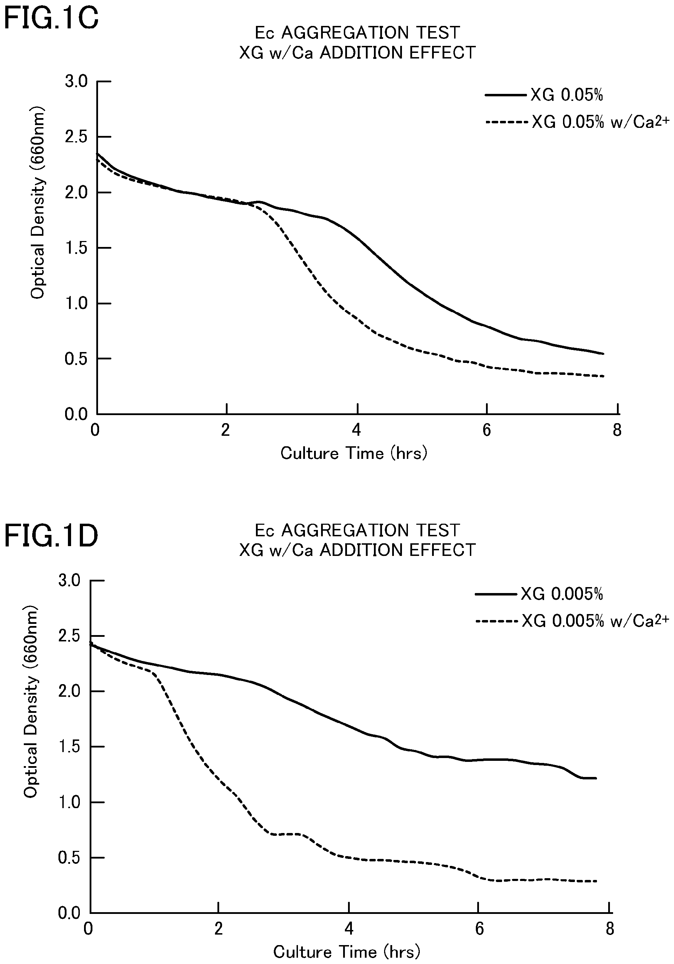

The results for using Ca salts in combination are presented in Figs. 1A to 1D and Table 3. It was found from Figs. 1A to ID that the use of Ca²⁺ in combination made it possible to lower the OD and shorten the aggregation time X, as compared with the case of using the polysaccharide alone at the same concentration.

The combined effect of divalent metal ions and polysaccharide carried out in the present test is an effect that cannot be obtained in the aggregation test of Japanese Patent No. 4294302, and is a finding that can be found only by the evaluation method described in the present specification.

**[Table 3]**

| Poly-Sacchride(PS) | w/Ca* | Conc.(%) | RT(hr) | Aggregation Rate (/h) |
|---|---|---|---|---|
| w/o PS | + | ND | 4.0 | 0.30 |
| Xanthan Gum | - | 0.050 | 4.0 | 0.15 |
| | + | 0.050 | 4.0 | 0.32 |
| | - | 0.005 | 4.0 | 0.14 |
| | + | 0.005 | 4.0 | 0.44 |
| Pullulan | - | 0.500 | 3.3 | 0.55 |
| | + | 0.500 | 2.5 | 0.76 |
| | - | 0.050 | 4.0 | 0.04 |
| | + | 0.050 | 4.0 | 0.43 |

| | | | | |
|---|---|---|---|---|
| w/Ca: added such that CaCl2 (10 mM). | | | | |

### Example 2: Preparation of Coated-Type Feed Additive

The core materials used were arabic gum (manufactured by Wako Pure Chemical Industries, Ltd.), pullulan (manufactured by Carbosynth Limited), and xanthan gum (manufactured by Sigma-Aldrich), and the coating agents used were hydrogenated rapeseed oil (melting point 67°C) and natural resin shellac. The powdered or granular core was sprayed with a predetermined amount of coating agent, liquefied by heating to a temperature higher than the melting point, to obtain a coated-type feed additive.

In the one-layer coating, 20 parts by mass of hydrogenated rapeseed oil was coated on 80 parts by mass of the core.

In the two-layer coating, 5 parts by mass of shellac was coated as the first layer (inner layer), and 17 parts by mass of hydrogenated rapeseed oil was coated as the second layer (outer layer), based on 77 parts by mass of the core.

### Example 3: Enteric Test

(Artificial Gastric Juice Treatment) To pure water produced using a pure water production device manufactured by Merck Millipore, 0.2% NaCl and 0.2% pepsin (from Porcine stomach Mucosa, 1:5,000, 2,500 unit/mg) were added to adjust the pH to 2, and then the coated-type feed additive prepared in Example 2 (the core was xanthan gum or arabic gum) was charged therein, followed by enzyme treatment at 37°C for 2 hours. The dissolution rate was measured by automatically and continuously measuring the optical density during this period. Note that the "2 hours" assumes the time from when the feed reaches the stomach of the chicken until it passes. (Artificial Intestinal Juice Treatment) After the artificial gastric juice treatment, 0.2% trypsin (from Porcine Pancreas, 1:5,000; 4,500 unit/mg) was added to adjust the pH to 6, followed by enzyme treatment at 37°C for 2 hours. The dissolution rate was measured by automatically and continuously measuring the optical density during this period. Note that the "2 hours" assumes the time from when the feed reaches the intestine of the chicken until it passes.

Note that the optical density in both treatments was measured at OD 190 nm using Biophoto-recorder TVS062CA manufactured by ADVANTEC. In addition, hydrochloric acid and sodium hydroxide were used as pH adjusters in both treatments.

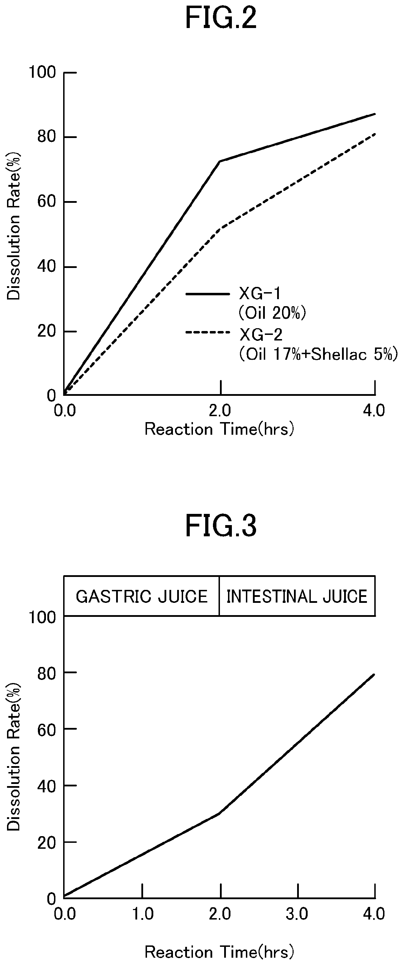

Figs. 2 and 3 illustrate the results. Fig. 2 reveals that, in the one-layer coating (XG-1), the dissolution rate at 2 hours after the start of gastric juice treatment exceeded 70%, whereas, in the two-layer coating (XG-2), the dissolution rate at 2 hours after the start of gastric juice treatment was suppressed to 50% or less, while 80% was exceeded in intestinal juice treatment. It was found from these results that the feed additive having the layer of hydrogenated rapeseed oil as the outer layer and the layer of shellac as the inner layer was superior in release control to the feed additive having only the layer of hydrogenated rapeseed oil as the coating agent. When the knowledge obtained for xanthan gum (XG) was utilized to also perform a two-layer coating on arabic gum, the dissolution rate at 2 hours after the start of gastric juice treatment was suppressed to 50% or less, while 80% was exceeded in intestinal juice treatment (Fig. 3). It was found from these results that, also for arabic gum, the feed additive having the layer of hydrogenated rapeseed oil as the outer layer and the layer of shellac as the inner layer was superior in release control to the feed additive having only the layer of hydrogenated rapeseed oil as the coating agent.

### Example 4-1: Chicken Growth Test Using Coated-Polysaccharide

The coated-type feed additive with a two-layer coating prepared in Example 2 (the core was arabic gum, pullulan) was added to the feed matrix having the composition presented in Table 4 so that the amount of the core material was 100 ppm to obtain a feed composition. In a flat poultry house, 25 neonate broiler chickens in 1 section were used, fed with the feed composition, and bred for 22 days at 0 to 21 days of age in triplicate to thereby evaluate the chicken body weight gain effect (BWG) and feed conversion ratio (FCR = Feed/BWG).

Note that a conventional antibiotic growth promoterotic (AGP), avilamycin, was used as a positive control. As avilamycin, a commercially available product (Surmax 200 (registered trademark) manufactured by ELANCO, uncoated) was used as it was.

The results are presented with a negative control of 100. Table 5 presents the results.

**[Table 4]**

| *Feed Composition | |
|---|---|
| Raw Material | Mixing Ratio, Mass% |
| Corn | 45.4 |
| Grain Sorghum | 10.0 |
| Soybean Meal | 30.0 |
| Corn Gluten Meal | 4.00 |
| Fishmeal (CP 65%) | 3.00 |
| L-Lysine Hydrochloride | 0.31 |
| DL-Methionine | 0.35 |
| L- Threonine | 0.12 |
| L-Arginine | 0.16 |
| Animal Oil and/or Fat | 3.49 |
| Dibasic Calcium Phosphate | 1.45 |
| Calcium Carbonate | 1.06 |
| Salt | 0.30 |
| Vitamin and Mineral Premix | 0.25 |
| Choline Chloride | 0.02 |
| L-Valine | 0.07 |
| Total | 100 |

**[Table 5]**

| | Addition [ppm] | 0-3W | |
|---|---|---|---|
| | | BWG[%] | FCR[%] |
| Control | 0 | 100 | 100 |
| Avilamycin(AGP) | 10 | 108 | 97 |
| Coated Arabic Gum | 100 | 106 | 98 |
| Coated pullulan | 100 | 105 | 99 |

From the results presented in Table 5, both coated arabic gum and coated pullulan exhibited the same body weight gain effect and feed conversion ratio as avilamycin, so that their effectiveness could be demonstrated.

### Example 4-2: Chicken Growth Test Using Coated-Polysaccharide - Ca Divalent Ion Synergistic Effect

The coated-type feed additive with a two-layer coating prepared in Example 2 (the core was pullulan) and the coated-type feed additive with a two-layer coating prepared in the same manner except that the core contained CaCl2 were added to the feed matrix having the composition presented in Table 4 so that the amount of the core material was the concentration presented in Table 6 to obtain a feed composition. The obtained feed composition was used to carry out a chicken growth test in the same manner as in Example 4-1. When fed, coated-PLN (100 ppm) and coated-PLN w/CaCl2 (10 ppm each for coated-PLN and CaCl2) were confirmed to improve body weight gain of 1.2% each.

Note that the coated-type feed additive containing CaCl2 in the core was mixed at pullulan:CaCl2·2H2O = 10:3 (mass ratio), and based on 100 parts by mass of the total amount thereof, 5 parts by mass of shellac and 17 parts by mass of hydrogenated rapeseed oil were coated in two layers in this order.

**[Table 6]**

| | Addition [ppm] | 0-3W | |
|---|---|---|---|
| | | BWG[%] | FCR[%] |
| NC | 0 | 100 | 100 |
| Enramycin (AGP) | 10 | 103 | 96 |
| Coated pullulan | 100 | 101 | 99 |
| Coated pullulan + CaC12 | 10 | 102 | 98 |

Coated pullulan alone had a weak effect in terms of body weight gain effect and feed conversion ratio, but the effect of body weight gain effect and feed conversion ratio could be demonstrated by adding only 1/10 of pullulan and adding calcium chloride (10 ppm).

### Example 4-3: Evaluation of Arabic Gum and Coated-Arabic Gum by Salmonella Infection Test in Chickens

The coated-type feed additive with a two-layer coating prepared in Example 2 (the core was arabic gum) was added to the feed matrix having the composition presented in Table 4 so that the amount of the core material was 100 ppm to obtain a feed composition. Broilers at 0 days of age were introduced into a breeding facility for infection test (6 broilers/repeat and 2 repeats/test group), then Salmonella typhimurium (ST) was orally administered, and the test feed was fed for 21 days to evaluate the body weight gain effect and feed conversion ratio.

Note that a conventional antibiotic growth promoterotic (AGP), avilamycin, was used as a positive control. As avilamycin, a commercially available product (Surmax 200 (registered trademark) manufactured by ELANCO, uncoated) was used as it was.

The results are presented with a negative control of 100. Table 7 presents the results.

**[Table 7]**

| Test Group | Addition [ppm] | 0-3 week | |
|---|---|---|---|
| | | BWG [%] | FCR [%] |
| Negative Control | - | 100% | 100% |
| Avilamycin (AGP) | 10 | 105% | 99% |
| Arabic Gum | 100 | 101% | 99% |
| Coated Arabic Gum | 100 | 106% | 98% |

As a result, uncoated gum arabic had a weak effect on body weight gain effect and feed conversion ratio, but coated gum arabic exhibited a body weight gain effect and feed conversion ratio improvement effect equivalent to or higher than those of avilamycin.

## Claims

1. A coated-type feed additive composition for improving body weight gain of livestock or feed conversion ratio for livestock comprising:
a core containing a polysaccharide having a property of aggregating gram-negative bacteria; and
a core coating agent.

2. A coated-type feed additive composition for improving body weight gain of livestock or feed conversion ratio for livestock according to claim 1, wherein
the polysaccharide is a polysaccharide containing, as a constituent sugar, at least one selected from the group consisting of mannose (Man), glucose (Glc), galactose (Gal), glucuronic acid (GlcA), mannuronic acid, and guluronic acid.

3. A coated-type feed additive composition for improving body weight gain of livestock or feed conversion ratio for livestock according to claim 1, wherein
the polysaccharide is at least one selected from the group consisting of pullulan, xanthan gum, guar gum, chitosan, carrageenan, arabic gum, pectin, carboxymethyl cellulose, methyl cellulose, hydroxypropyl cellulose, hydroxypropyl methylcellulose, dextran, chondroitin, tara gum, locust bean gum, alginates (sodium salts, potassium salts, calcium salts, or ammonium salts), alginic acid esters, and mixtures thereof.

4. A coated-type feed additive composition for improving body weight gain of livestock or feed conversion ratio for livestock according to claim 1, wherein
the polysaccharide is a culture product of Xanthomonas campestris, Aureobasidium pullulans, Lipomyces starkeyi, or brown algae (Phaeophyta).

5. A coated-type feed additive composition for improving body weight gain of livestock or feed conversion ratio for livestock according to any one of claims 1 to 4, wherein
the coating agent contains hydrogenated vegetable oil.

6. A coated-type feed additive composition for improving body weight gain of livestock or feed conversion ratio for livestock according to claim 5, wherein
the hydrogenated vegetable oil is a hydrogenated rapeseed oil, hydrogenated linseed oil, hydrogenated safflower oil, hydrogenated sunflower oil, hydrogenated soybean oil, hydrogenated corn oil, hydrogenated peanut oil, hydrogenated cottonseed oil, hydrogenated sesame oil, hydrogenated rice oil, hydrogenated olive oil, hydrogenated palm oil, hydrogenated palm kernel oil, or hydrogenated coconut oil.

7. A coated-type feed additive composition for improving body weight gain of livestock or feed conversion ratio for livestock according to any one of claims 1 to 6, wherein
the polysaccharide is pullulan, xanthan gum, or arabic gum, and
the coating agent is hydrogenated rapeseed oil and/or shellac.

8. A coated-type feed additive composition for improving body weight gain of livestock or feed conversion ratio for livestock according to claim 7, wherein
a coating layer formed by the coating agent has a two-layer structure including a layer formed of hydrogenated rapeseed oil and a layer formed of shellac.

9. A coated-type feed additive composition for improving body weight gain of livestock or feed conversion ratio for livestock according to claim 8, wherein
the layer formed of shellac is in contact with the core, and the layer formed of hydrogenated rapeseed oil is formed thereon.

10. A coated-type feed additive composition for improving body weight gain of livestock or feed conversion ratio for livestock according to any one of claims 1 to 9, wherein
the core further contains a salt of a divalent inorganic cation.

11. A coated-type feed additive composition for improving body weight gain of livestock or feed conversion ratio for livestock according to claim 10, wherein
the salt of a divalent inorganic cation is a salt of Mg, Ca, or Fe.

12. A coated-type feed additive composition for improving body weight gain of livestock or feed conversion ratio for livestock according to claim 11, wherein
the salt of a divalent inorganic cation is a salt of Ca.

13. A feed comprising:
a coated-type feed additive composition for improving body weight gain of livestock or feed conversion ratio for livestock according to any one of claims 1 to 12.

14. Use of a coated agent comprising a core containing a polysaccharide having a property of aggregating gram-negative bacteria and a core coating agent, as an improver for body weight gain of livestock or feed conversion ratio for livestock.
